(19) Europäisches Patentamt
European Patent Office
Office européen des brevets

(11) Veröffentlichungsnummer: **0 243 300**
**A2**

# (12) EUROPÄISCHE PATENTANMELDUNG

(21) Anmeldenummer: **87810150.0**

(22) Anmeldetag: **16.03.87**

(51) Int. Cl.$^4$: **G 01 N 21/64**

(30) Priorität: **21.03.86 CH 1141/86**

(43) Veröffentlichungstag der Anmeldung:
**28.10.87 Patentblatt 87/44**

(84) Benannte Vertragsstaaten:
**AT BE CH DE ES FR GB GR IT LI LU NL SE**

(71) Anmelder: **CIBA-GEIGY AG**
**Klybeckstrasse 141**
**CH-4002 Basel (CH)**

(72) Erfinder: **Koch, Herbert Anton, Prof. Dr.**
**Im Diepental 34**
**D-4000 Düsseldorf 13 (DE)**

## (54) Nachweis von Pilzen.

(57) Die Erfindung betrifft ein Verfahren zum Nachweis von Pilzen und Algen in tierischen und menschlichen Geweben und Körperflüssigkeiten, Präparate enthaltend einen für das Verfahren geeigneten optischen Aufheller und eine Test-Packung zur Ausübung der Verfahrens. Die zu untersuchenden Gewebeteile oder Proben von Körperflüssigkeiten werden mit einem optischen Stilben-Aufheller behandelt und mit einem Fluoreszenz-Mikroskop betrachtet. Die Erfindung ermöglicht eine schnelle und zuverlässige Diagnose von Pilzinfektionen.

EP 0 243 300 A2

**Beschreibung**

Nachweis von Pilzen

Die Erfindung betrifft ein Verfahren zum Nachweis von Pilzen und Algen in tierischen und menschlichen Geweben und Körperflüssigkeiten, Präparate enthaltend einen für das Verfahren geeigneten optischen Aufheller und eine Test-Packung zur Ausübung des Verfahrens.

Eine genaue Diagnose der Ursache einer Infektion bei Tier und Mensch ist von entscheidender Bedeutung für die Wahl der einzuschlagenden Behandlung. Die beste Methode für eine zuverlässige Diagnose besteht darin, den Erreger der Infektion aus klinischen Präparaten zu isolieren und morphologisch zu identifizieren. Ein solcher Idealfall wird jedoch selten erreicht, weil die Isolierung aufwendig und die Identifikation häufig unsicher ist. Schon eine vorläufige Diagnose von Pilzinfektionen erfordert umfangreiche Untersuchungen von Gewebeproben oder Körperflüssigkeiten, beispielsweise serologische Tests mit Hilfe einer Reihe von verschiedenen Antikörpern, die gewisse Antigene von Pilzen selektiv erkennen.

Eine schnelle und zuverlässige Diagnose, die die umständlichen und zeitaufwendigen serologischen Tests ersetzen oder zumindest auf jene Fälle beschränken könnte, in denen genaue Kenntnisse über die Gattung des Pilzerregers der Infektion nötig sind, entspricht einem weit verbreiteten Bedürfnis. Eine solche Schnelldiagnose ist beispielsweise von Bedeutung bei der Untersuchung von Operationsmaterial, gynäkologischen Abstrichen zur Bestimmung eines durch Pilze bedingten Ausflusses, dermatologischen Abstrichen bei Verdacht auf Dermatomykosen, pädiatrischem Abstrich- und anderem Untersuchungs material von Neugeborenen und geschwächten Kindern mit Allgemeininfektionen und Verdacht auf Mykosen auf den Schleimhäuten, beispielsweise Candida-bedingtem Soor auf Mund- und Rachenschleimhäuten und Speise- und Luftröhre bei Leukämien und anderen Tumorerkrankungen im Kindesalter. Weiter ist eine Schnelldiagnose von Bedeutung in der internistischen, onkologischen und chirurgischen Praxis bei der Untersuchung von Gewebematerial von Patienten mit Verdacht auf opportunistische Pilzinfektionen, z.B. bei AIDS-Patienten zur Erkennung insbesondere von Candidiasis in Form einer Oesophagitis und Bronchitis, bei Patienten unter zytostatischer Behandlung von Tumorerkrankungen und bei Patienten unter Behandlung mit Antibiotika und/oder Immunsuppressiva, ferner bei Verdacht auf Mykosen, insbesondere Candidiasis, in Form eines Soor auf Schleimhäuten, in der Geriatrie bei der Untersuchung abwehrgeschwächter Patienten mit generalisierten Mykosen auf der Haut bzw. auf den Schleimhäuten, und bei Verdacht auf Haut- oder Organinfektionen durch Algen, z.B. Protothecose.

Eine Diagnose von Pilzerkrankungen ist auch von Bedeutung bei der Haltung von Nutz- und Haustieren, beispielsweise bei der Untersuchung von Mastitis bei Rindern und bei Pilzerkrankungen z.B. von Hunden, Ziervögeln und seltenen und kostbaren Zootieren. Eine einfache und zuverlässige Methode zur Erkennung von Pilzen ist ferner wichtig bei der Prüfung potentiell antifungischer Therapeutika an Labortieren, z.B. Mäusen und Ratten, mit kunstlich erzeugten Pilzinfektionen.

Mit der vorliegenden Erfindung wird nun eine solche schnelle und zuverlässige Diagnose von Pilzinfektionen ermöglicht. Die Erfindung betrifft ein Verfahren zum Nachweis von Pilzen und Algen, dadurch gekennzeichnet, dass tierisches oder menschliches Gewebe oder Körperflüssigkeiten mit einem optischen Stilben-Aufheller mit mindestens 4 Sulfogruppen oder einem Salz davon behandelt und mit einem Fluoreszenz-Mikroskop untersucht werden.

Optische Aufheller sind farblose oder nur eine schwach sichtbare Färbung aufweisende organische Verbindungen, die ultraviolettes Licht, beispielsweise einer Wellenlänge zwischen 340 und 400 nm, absorbieren und diese aufgenommene Energie in Form von sichtbarem Licht, beispielsweise einer Wellenlänge zwischen zwischen 400 und 440 nm, wieder abgeben. Gute optische Aufheller zeichnen sich durch eine hohe Quantenausbeute aus. Optische Aufheller gehören beispielsweise der Verbindungsklasse der Stilbene, Coumarine und Carbostyril-Verbindungen, 1,3-Diphenyl-2-pyrazoline, Naphthalimide, Benzoxazolylverbindungen oder anderer heteroaromatisch substituierter Aromaten oder Ethylenverbindungen an und weisen immer ein konjugiertes $\pi$-Elektronensystem auf.

Die optischen Aufheller der vorliegenden Erfindung gehören zur Verbindungsklasse der substituierten Stilbene und tragen mindestens 4, beispielsweise 4 bis 6 Sulfogruppen. Diese Sulfogruppen vermitteln dem optischen Aufheller eine gute Wasserlöslichkeit. Die Herstellung der optischen Stilben-Aufheller mit mindestens 4 Sulfogruppen und ihrer Salze ist bekannt und ist beispielsweise in Uebersichtsartikeln und Fachbüchern beschrieben, z.B. in "Fluorescent Whitening Agents" (Herausgeber R. Anliker und G. Müller), Georg Thieme Verlag, Stuttgart 1975.

Beispiele für optische Stilben-Aufheller mit mindestens 4 Sulfogruppen sind Verbindungen der Formel

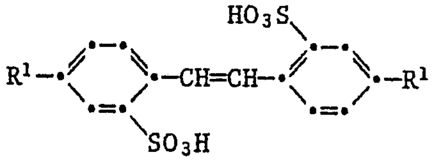

(I),

worin $R^1$ ein aromatischer oder heteroaromatischer Substituent, der mindestens eine Sulfogruppe trägt, beispielsweise ein substituierter Phenylrest, Pyridylrest oder Triazolylrest, z.B. ein durch Sulfo substituierter 4-Phenyl-1,2,3-triazol-2-ylrest, oder eine durch einen aromatischen oder heteroaromatischen Rest mit mindestens einer Sulfogruppe substituierte Aminogruppe bedeutet, beispielsweise substituiertes Triazinylamino, z.B. 2,4-disubstituiertes 1,3,5-Triazin-6-ylamino mit mindestens einer Sulfogruppe, und die beiden Reste $R^1$ gleich oder verschieden sein können, ferner Verbindungen der Formel

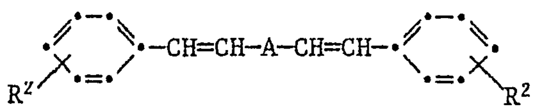

(II),

worin A ein durch Sulfogruppen substituierter 1,4-Phenylen- oder 4,4'-Biphenylylen-Rest und $R^2$ Sulfo, Hydroxy, Niederalkoxy, z.B. Methoxy, Hydroxyniederalkoxy, z.B. 2-Hydroxyethoxy, Halo, z.B. Chlor, Cyano, Carboxy, Niederalkoxycarbonyl, z.B. Methoxycarbonyl oder Ethoxycarbonyl, oder Carbamoyl bedeutet oder die Bedeutungen von $R^1$ hate und die Reste $R^2$ gleich oder verschieden sein und auch mehrfach vorkommen können.

Niederalkyl und der Niederalkylrest in einer Niederalkoxygruppe hat 1 bis 7, bevorzugt 1 bis 4, Kohlenstoffatome und ist beispielsweise Methyl, Ethyl, Propyl, Isopropyl oder Butyl. Bevorzugte Substituenten von Phenyl-, Pyridyl-, Triazolyl- oder Triazinylgruppen sind beispielsweise die weiter unten unter $R^3$ genannten Reste.

Salze sind insbesondere wasserlösliche Salze, z.B. Alkalimetall-, wie Natrium- oder Kaliumsalze, aber auch Ammoniumsalze.

Bevorzugt im Verfahren der vorligenden Erfindung sind optische Stilben-Aufheller der Formel

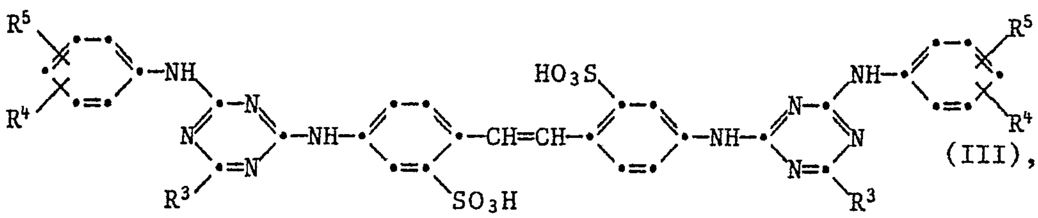

(III),

worin $R^3$ Hydroxy, Niederalkoxy, z.B. Methoxy, Hydroxyniederalkoxy, z.B. 2-Hydroxyethoxy, Niederalkoxyniederalkoxy, z.B. 2-Methoxyethoxy, Niederalkylamino, z.B. Methylamino, Diniederalkylamino, z.B. Dimethylamino, Hydroxyniederalkylamino, z.B. 2-Hydroxyethylamino oder 2-Hydroxypropylamino, Niederalkyl-hydroxyniederalkylamino, z.B. Methyl-2-hydroxyethyl-amino, Di(hydroxyniederalkyl)-amino, z.B. Di(2-hydroxyethyl)amino oder Di(2-hydroxypropyl)amino, Niederalkoxyniederalkylamino, z.B. 2-Methoxyethylamino, Niederalkyl-niederalkoxyniederalkyl-amino, z.B. Methyl-2-methoxyethylamino, Di(niederalkoxyniederalkyl)amino, z.B. Di(2-methoxyethyl)-amino, Morpholino oder Phenylamino, das gegebenenfalls im Phenylring durch Sulfogruppen substituiert ist. z.B. Anilino, 2-, 3- oder 4-Sulfophenylamino oder 2,5-Disulfophenylamino, $R^4$ Sulfo, z.B. 2-3- oder 4-Sulfo, und $R^5$ Wasserstoff, Sulfo, z.B. 4- oder 5-Sulfo, oder ein annellierter Phenylring, z.B. 2,3-, 3,4-5,6-Benzo, bedeuten und jeweils die beiden Reste $R^3$, $R^4$ und $R^5$ gleich oder verschieden sein können, ferner die davon abgeleiteten, wasserlöslichen Salze, z.B. Alkalisalze, wie Natrium- und Kaliumsalze.

Besonders bevorzugt im Verfahren der vorliegenden Erfindung sind optische Stilben-Aufheller der Formel III, worin $R^3$ Hydroxyniederalkylamino, z.B. 2-Hydroxyethylamino, Di(hydroxyniederalkyl)amino, z.B. Di(2-hydroxyethyl)amino, oder durch Sulfo substituiertes Phenylamino, z.B. 3-Sulfophenylamino, $R^4$ Sulfo, z.B. 2-, 3- oder 4-Sulfo, und $R^5$ Wasserstoff, Sulfo, z.B. 5-Sulfo, oder ein annellierter Phenylring, z.B. 2,3-Benzo, bedeuten, und ihre gut wasserlöslichen Salze, z.B. Natriumsalze.

In erster Linie betrifft die vorliegende Erfindung ein Verfahren unter Verwendung der Verbindung der Formel III, worin $R^3$ Di(2-hydroxyethyl)amino oder 3-Sulfophenylamino, $R^4$ 3-Sulfo und $R^5$ Wasserstoff bedeuten, und

ihrer wasserlöslichen Salze, bevorzugt des Natriumsalzes.

Der ganz besonders bevorzugte optische Stilben-Aufheller ist bekannt unter dem Namen Diäthanol und trägt den vollständigen Namen 4,4′-Bis[2-di(2-hydroxyethyl)amino-4-(3-sulfophenylamino)1,3,5-triazin 6-ylamino]-stilben-2,2′-disulfonsäure Natriumsalz. Die Verbindung kann beispielsweise nach der in U.S. Patent 3,406,118 beschriebenen Art und Weise hergestellt werden.

Tierisches oder menschliches Gewebe, das mit dem vorliegenden Verfahren zum Nachweis von Pilzen und Algen angefärbt werden kann, kann von beliebigen Organen oder Körperteilen stammen und wird beispielsweise in Form von Mikrotom-Gewebeschnitten von gefriergetrocknetem Gewebematerial oder von entwässertem, in Paraffin eingelegtem Gewebe oder in Form von Abklatsch- oder Abstrichpräparaten untersucht. Körperflüssigkeiten, in denen mit dem vorliegenden Verfahren Pilze und Algen nachgewiesen werden können, sind beispielsweise Blut, Sputum, Bronchialsekret, Lymphflüssigkeit, Cerebrospinalflüssigkeit und andere Punktate, Schweiss, Tränenflüssigkeit, Augeninnenflüssigkeit, Urin oder Stuhl.

Besonders bevorzugt ist ein Verfahren zum Nachweis von Pilzen und Algen in menschlichem Gewebe oder Körperflüssigkeiten.

Die Gewebeschnitte oder auf andere Weise zubereiteten Präparate, z.B. Abstriche, Abklatschpräparate oder aus Körperflüssigkeiten isolierten festen Bestandteile, werden bevorzugt mit einer wässrigen Lösung enthaltend 0,05 bis 5 %, bevorzugt 0,1-2%, des optischen Aufhellers, beispielsweise in Wasser, in einer Salzlösung, z.B. in gepufferter physiologischer Kochsalzlösung, oder in einer histologischen Fixierlösung, z.B. in 10% wässriger Formaldehydlösung oder einer wässrig-alkoholischen Lösung, während wenigen Minuten, beispielsweise 2 bis 30 Minuten, bei Raumtemperatur oder leicht erhöhter Temperatur, z.B. bis zu 50°C, behandelt. Ueberschüssiger optischer Aufheller wird gegebenenfalls weggewaschen, beispielsweise mit Wasser oder einer Salzlösung, z.B. gepufferter physiologischer Kochsalzlösung. Wenn erwünscht werden die zu untersuchenden Präparate anschliessend mit einem der üblichen Farbstoffe, die zum Einfärben von Gewebeschnitten oder Zellbestandteilen geeignet sind, beispielsweise mit Methylenblau, Toluidinblau, Fuchsin, Methylviolett oder degleichen, vorzugsweise aber mit Evans Blue, gegengefärbt, um die Kontrastwirkung zu erhöhen.

Die derart behandelten Präparate werden unter einem Mikroskop mit sichtbarem Blaulicht oder mit UV-Licht, bevorzugt im Bereich von 340 bis 450 nm, im Auflichtverfahren betrachtet. Die dabei zu beobachtende grünliche Fluoreszenzfarbe charakteristischer Strukturen von Pilzen oder Algen lässt das Vorhandensein solcher Erreger sofort erkennen. Aus der Morphologie der angefärbten Pilzbestandteile ist es in den meisten Fällen möglich, auf die Gattung und Art des Pilzes Rückschlüsse zu ziehen. Wenn erwünscht können die unter dem Mikroskop sichtbaren Bilder photographisch mit den üblichen, im Handel erhältlichen, farbempfindlichen Filmen festgehalten werden.

Das beschriebene erfindungsgemässe Verfahren zeichnet sich durch einfaches, zeitsparendes Vorgehen und grosse Zuverlässigkeit aus und ist damit andern Verfahren zur Diagnose von Pilzinfektionen klar überlegen. Gegenüber klassischen Anfärbemethoden, beispielsweise der normalerweise verwendeten Gomori-Methanamin-Silber-Färbung oder Periodsäure-Schiff-Färbung weist das erfindungsgemässe Verfahren den Vorteil auf, dass selektiv Pilze angefärbt werden, die Fluoreszenzfärbung sich gegen den ungefärbten Hintergrund besser abhebt und das Verfahren in weniger als 2 Stunden zuverlässige Ergebnisse liefert, während klassische Färbemethoden 24 Stunden und mehr benötigen. Einer Anfärbung mit Blankophor® BA oder Calcofluor® white M2R new, beides Triazinylaminostilben-Aufheller mit nur 2 Sulfogruppen, die schon zur Anfärbung von tierischen und menschlichem Gewebe empfohlen worden sind, ist das erfindungsgemässe Verfahren klar überlegen, weil selektiv alle Pilze und Algen angefärbt werden, während die genannten Aufheller verschiedene Pilze nicht oder nur schlecht anfärben und angefärbte Pilze im Gewebe nicht gut erkennbar sind.

Zur Ausübung des Verfahrens werden nur leicht zugängliche Reagentien und ein einfaches Fluoreszenz-Mikroskop benötigt, wie es in einem mikrobiologischen oder medizinischen Laboratorium üblicherweise zur Verfügung steht.

Positive Fluoreszenz mit dem bevorzugten wasserlöslichen optischen Aufheller Diäthanol wird bei sämtlichen Mykosen beobachtet, beispielsweise bei Lobo's Mykose der Haut, Blastomykose der Lunge, Trichosporose der Milz, Candidiasis der Niere, Histoplasmose der Lunge, Aktinomykose der Leber, Aspergillose der Niere, ferner Adiaspiromykose, Coccidioidomykose, Cryptococcose, Rhinosporidiose, Madura-Mycetoma, und vielen andern. Gleichermassen können isolierte und kultivierte Pilze angefärbt werden, beispielsweise die zum Stamm der Eumycota gehörenden Vertreter, z.B. Zygomyceten, Endomyceten, Ascomyceten oder Deuteromyceten (Fungi imperfecti), insbesondere Blastomyces dermatidis, Candida albicans, Aspergillus fumigatus, Sporotrichum schenckii, Trichophyton mentagrophytes, Trichophyton quinckeanum, Microsporum canis, Torulopsis glabrata, und andere, ferner Algen, z.B. Prototeca. Im Gegensatz dazu wird überraschenderweise bei Bakterien und Parasiten keine Anfärbung beobachtet, insbesondere sind Staphylokokken, z.B. Staph. aureus K 1326, Streptokokken, z.B. Strept. faecalis, Salmonellen, z.B. Salmonella K 1385, Coli-Bakterien, z.B. Escherichia coli K 1322, Pseudomonaden, z.B. Pseudomonas K 1407, und Mykobakterien, z.B. Mycobact. smegmatis nicht anfärbbar. Ebenfalls bemerkenswert ist, dass wichtige pathogene, nicht zu den Pilzen gehörende Erreger, z.B. Pneumocystis carinii, Giardia lamblia, Leishmania donovani, Leish. chagasi und Leish. infantum, nicht angefärbt werden.

Die Erfindung betrifft ferner Präparate enthaltend einen zur Ausübung des Verfahrens geeigneten optischen Aufheller. Solche Präparate sind beispielsweise wässrige Lösungen der oben genannten optischen Stilben-Aufheller mit mindestens 4 Sulfogruppen, ferner feste Mischungen, z.B. pulverige oder körnige

Mischungen.

Erfindungsgemässe wässrige Lösungen enthalten beispielsweise 0,05 % bis 5 %, bevorzugt 0,1 - 2 %, z.B. 1 %, des optischen StilbenAufhellers und gewünschtenfalls weitere Stoffe, beispielsweise Salze, z.B. Natriumchlorid, Kaliumchlorid, Natriumsulfat, Natriumoder Kaliumdihydrogenphosphat, Dinatrium- oder Dikaliumhydrogenphosphat oder andere Puffersalze, organische Pufferverbindungen, z.B. N-2-Hydroxyethyl-piperazin-N'-2-ethansulfonsäure, 3-Morpholinopropansulfonsäure, N-Tris(hydroxymethyl)methylglycin oder Tris(hydroxymethyl)aminomethan und ihre Alkalimetallsalze, z.B. Natriumsalze, beziehungsweise Säureadditionssalze, z.B. Hydrochloride, denaturierende und/oder lösungsvermittelnde organische Lösungsmittel, z.B. Methanol oder Ethanol, und fixierende Stoffe, z.B. Formaldehyd, ferner gewünschtenfalls Streckmittel, z.B. Harnstoff, Netzmittel, z.B. aliphatische oder aromatische Sulfonate, oder Komplexiermittel für Calciumionen, z.B. Ethylendiamin-tetraessigsäure Natriumsalz. Die Konzentration an optischem Stilben-Aufheller in der wässrigen Lösung kann auch mehr als 5 %, beispielsweise bis zu 20 %, betragen. Solche Lösungen müssen vor der Anwendung im erfindungsgemässen Verfahren mit Wasser verdünnt werden.

Erfindungsgemässe feste Mischungen sind Mischungen der optischen Stilben-Aufheller mit den obengenannnten Salzen, z.B. Natriumchlorid, Kaliumchlorid und/oder Puffersalzen, wie Natriumdihydrogenphosphat und Dinatriumhydrogenphosphat, oder mit organischen, festen Pufferverbindungen, z.B. Tris(hydroxymethyl)aminomethan und dem entsprechenden Hydrochlorid. Diese Mischungen müssen vor der Anwendung im erfindungsgemässen Verfahren in Wasser gelöst werden.

Bevorzugte Präparate sind wässrige Lösungen der weiter oben als bevorzugt genannten optischen Stilben-Aufheller, z.B. von 4,4'-Bis-[2-di(2-hydroxyethyl)amino-4-(3-sulfophenylamino)-1,3,5-triazin-6-ylamino]-stilben-2,2'-disulfonsäure Natriumsalz, bekannt als Diäthanol. Besonders bevorzugt sind solche Lösungen enthaltend 0,1 - 2 %, z.B. 1%, des bevorzugten Aufhellers in destilliertem Wasser oder in phosphatgepufferter physiologischer Kochsalzlösung.

Die erfindungsgemässen Präparate werden nach an sich bekannten Lösungs-, Misch-, Mahl-, Granulier- und/oder Lyophilisierungs-Verfahren hergestellt.

Die Erfindung betrifft ebenfalls Test-Packungen zur Durchführung des erfindungsgemässen Verfahrens. Solche Test-Packungen enthalten beispielsweise einen optischen Stilben-Aufheller mit mindestens 4 Sulfogruppen als solchen oder in Form eines oben beschriebenen erfindungsgemässen Präparats, Farbstoffe oder Färbelösungen zur Gegenfärbung, Puffersubstanzen oder Pufferlösungen, dazu Hilfsmittel wie Pipetten, Objektträger und Deckgläser für Mikroskope, eine Anleitung zur Durchführung des erfindungsgemässen Verfahrens, Vergleichsproben und dergleichen.

Erfindungsgemässe Test-Packungen können zusätzlich serologische Test-Lösungen enthalten, beispielsweise polyklonale oder monoklonale Antikörper-Lösungen, mit denen eine erste vorläufige Bestimmung einer Pilzgattung bei der Anfärbung mit dem optischen Stilben-Aufheller noch zusätzlich bestätigt werden kann. Solche polyklonalen ind monoklonalen Antikörper, die gegen Marker-Antigene der verschiedenen Pilz-Gattungen gerichtet sind und spezifisch an die entsprechenden Pilze binden, sind bekannt oder können nach bekannten Verfahren hergestellt werden. Bevorzugt werden fluoreszenzmarkierte Antikörper, beispielsweise mit Rhodamin- oder Fluorescein-isothiocyanat markierte Antikörper, verwendet, um Antigen-Antikörper-Reaktionen leicht im Fluoreszenz-Mikroskop sichtbar machen zu können. Die Test-Pakkungen können auch Antikörper-Lösungen enthalten, die spezifisch nur mit gewissen Bakterien oder gewissen Protozoen reagieren. Bevorzugt werden dann solche fluoreszenzmarkierte Antikörper verwendet, deren Fluoreszenzfärbung von der Färbung mit dem erfindungsgemässen Stilben-Aufheller unterschieden werden kann, beispielsweise mit Rhodamin-isothiocyanat markierte Antikörper, damit in einem Arbeitsgang Pilze und Bakterien beziehungsweise Protozoen nebeneinander nachgewiesen werden können.

Die nachfolgenden Beispiele illustrieren die Erfindung, schränken jedoch deren Umfang in keiner Weise ein.

Beispiel 1: Anfärbung von Pilzen in Gewebeschnitten

Gewebestücke, beispielsweise Biopsiematerial, werden in üblicher Weise in Kapseln in Paraffin eingebettet, das Paraffin ausgehärtet und die Blöcke im Mikrotom auf eine Schichtdicke von 3-4 µm geschnitten. Die Paraffinschnitte werden auf Objektträger aufgebracht, im Wasserbad gestreckt, getrocknet und das Paraffin dreimal mit Xylol, dann mit Alkohol, Alkohol-Wasser-Gemischen, Wasser und schliesslich phosphatgepufferter physiologischer Kochsalzlösung (PBS) ausgewaschen. Anstelle von Paraffinschnitten können auch Mikrotom-Schnitte von ca. 10 µm Dicke von tiefgefrorenem Gewebematerial aufgebracht und mit PBS konditioniert werden.

Die Gewebeschnitte werden mit einer Lösung von 1 % Diäthanol in PBS, pH 7 getränkt. Nach 10 bis 30 Minuten werden die Schnitte mit PBS gespült, dann mit einer 0,2 % Lösung von Evans Blue in PBS behandelt und nach 1/2 Minuten erneut mit PBS gewaschen. Die Schnitte werden in Wasser auf Objektträgern eingedeckelt und mit Nagellack versiegelt. Die Gewebeschnitte werden in einem Olympus® BHTU Mikroskop mit Fluoreszenzlicht im Auflichtverfahren betrachtet. Zellwand und Zellplasma von Pilzen sind grünlich oder grünlich-gelblich fluoreszierend angefärbt, während der Hintergrund eine hellbräunliche Färbung annimmt. Dadurch kommt eine gute Kontrastwirkung zustande. Die Mikroskopaufnahmen werden mit einer Leica-Kleinbildkamera mit Kodak Farbfilm ASA 400 photographiert.

Beispiel 2: Anfärbung von Material aus Kulturbrühen oder Körperflüssigkeiten

Zu untersuchendes Material wird auf einen Objektträger ausgestrichen, luftgetrocknet, mit einer Flamme hitzefixiert und 1 Minute mit Methanol überschichtet. Der Objektträger wird mit einer wässrigen Lösung von 0,1 % Diäthanol überschichtet. Nach 5 Minuten wird die Färbelösung abgegossen und der Objektträger mit Wasser gespült und luftgetrocknet. Die Probe wird mit einem Deckglas abgedeckt und in einem Dialux-20® Mikroskop bei 40-facher Vergrösserung und Auflicht-UV-Beleuchtung mit Filter H2 (Wellenlänge 390-490 nm) betrachtet.

Zur Verbesserung der Kontrastwirkung wird die mit dem Aufheller eingefärbte Probe vorteilhafterweise vor dem Trocknen 1/2 Minuten lang mit einer 0,2 % wässrigen Lösung von Evans Blue behandelt und mit Wasser gespült.

Beispiel 3: Prüfung von optischen Aufhellern als Anfärbemittel für Pilze

Candida albicans K 1133, Aspergillus fumigatus K 76 Microsporum canis K 240 werden auf Mycophilagar gezüchtet, für die Testreihe in physiologischer Kochsalzlösung suspendiert und zu gleichen Teilen mit einer Suspension zermalmter Mäusenieren gemischt. Jeweils 1 Tropfen dieser Mischung wird auf einen Objektträger gegeben, an der Luft getrocknet, zum Fixieren dreimal durch eine Flamme gezogen, 1 Minute lang mit Methanol überschichtet und mit destilliertem Wasser gespült. Die fixierte Pilzprobe wird mit einer 1 % wässrigen Lösung des zu untersuchenden optischen Aufhellers während 5 Minuten überschichtet, mit phosphatgepufferter physiologischer Kochsalzlösung (PBS) gespült, 1/2 Minuten mit einer 0,2 % Lösung von Evans Blue in PBS überschichtet, mit PBS gespült und mit einem Deckglas abgedeckt. Die Probe wird in einem Dialux-20® Mikroskop bei 40-facher Vergrösserung und Auflicht-UV-Beleuchtung (390-490 nm) betrachtet.

Die Leuchtkraft der Fluoreszenz der Pilze in Beziehung zur Anfärbung des organzelligen Hintergrunds wird mit einer willkürlichen Skala bewertet. Pro Pilz werden 2-3 Versuche durchgeführt und die Resultate gemittelt. Dabei werden folgende Resultate erhalten:

Sehr gute Anfärbung mit:

4,4′-Bis[2-di(2-hydroxyethyl)amino-4(3-sulfophenylamino)-1,3,5-triazin-6-ylamino]-stilben-2,2′-disulfonsäure Natriumsalz (Diäthanol) und

4,4′-Bis-[2,4-di(3-sulfophenylamino)-1,3,5-triazin-6-ylamino]-stilben-2,2′-disulfonsäure Natriumsalz.

Gute Anfärbung mit:

4,4′-Bis-[2-di(2-hydroxyethyl)amino-4-(4-sulfo-1-naphthylamino)-1,3,5-triazin-6-ylamino]-stilben-2,2′-disulfonsäure Natriumsalz,

4,4′-Bis-[2-di(2-hydroxyethyl)amino-4-(2,5-disulfophenylamino)-1,3,5-triazin-6-ylamino]-stilben-2,2′-disulfonsäure Natriumsalz,

4,4′-Bis-[2-(2-hydroxyethyl)amino-4-(3-sulfophenylamino)-1,3,5-triazin-6-ylamino]-stilben-2,2′-disulfonsäure Natriumsalz und

4,4′-Bis-[2-di(2-hydroxyethyl)amino-4-(4-sulfophenylamino-1,3,5-triazin-6-ylamino]-stilben-2,2′-disulfonsäure Natriumsalz.

Mittlere Anfärbung mit:

4-[2-Di(2-hydroxyethyl)amino-4-methoxy-1,3,5-triazin-6-ylamino]-4′-[2-di(2-hydroxyethyl)amino-4-(2-sulfoethylamino)-1,3,5-triazin-6-ylamino]-stilben-2,2′-disulfonsäure Natriumsalz,

4,4′-Bis-[2-morpholino-4-(2,5-disulfophenylamino)-1,3,5-triazin-6-ylamino]-stilben-2,2′-disulfonsäure Natriumsalz,

4,4′-Bis-[2-di(2-hydroxypropyl)amino-4-(3-sulfophenylamino)-1,3,5-triazin-6-ylamino]-stilben-2,2′-disulfonsäure Natriumsalz und

4,4′-Bis-[2-di(2-hydroxypropyl)amino-4-(4-sulfophenylamino)-1,3,5-triazin-6-ylamino]-stilben-2,2′-disulfonsäure Natriumsalz.

Stilbenaufheller mit nur 2 Sulfogruppen oder mit Carboxy- und Ammoniumgruppen anstelle der Sulfogruppen und Stilbenaufheller ohne den 2,4-substituierten 1,3,5-Triazin-6-ylamino-Rest zeigen weniger gute oder gar keine Anfärbung.

Beispiel 4: Diäthanol in phosatgepufferter physiologischer Kochsalz-Lösung

10,0 g 4,4′-Bis]2-di(2-hydroxyethyl)amino-4-(3-sulfophenylamino)-1,3,5-triazin-6-ylamino]-stilben-2,2′-disulfonsäure Natriumsalz (Diäthanol, Kochsalz-frei), 0,2 g Kaliumchlorid, 8,0 g Natriumchlorid, 1,44 g Dinatriumhydrogenphosphat-Dihydrat und 0,2 g Kaliumdihydrogenphosphat werden in 1 ℓ destilliertem Wasser gelöst.

Die gleiche Lösung kann auch mit Kochsalz (13 %) und Wasser (5-10 %) enthaltender Diäthanol-Rohware hergestellt werden, wobei die Mengen entsprechend angepasst werden.

Beispiel 5: Test-Packung

Eine Test-Packung zur Anfärbung von Pilzen für Grossverbraucher enthält:

Schraubdeckelflasche enthaltend 250 ml einer 1%igen Lösung von Diäthanol in phosphatgepufferter Kochsalz-Lösung,

Schraubdeckelflasche enthaltend 250 ml einer 0,2%igen Lösung von Evans Blue in phosphatgepufferter Kochsalz-Lösung,

Anleitung zur Durchführung des Färbeverfahrens.

Beispiel 6: Test-Packung

Eine Test-Packung zur Anfärbung von Pilzen für Kleinverbraucher enthält:
20 Aufreisspackungen aus polyethylenbeschichteter Aluminiumfolie enthaltend je 1 ml einer 1%igen Lösung von Diäthanol in phosphatgepufferter Kochsalzlösung,
20 Aufreisspackungen aus polyethylenbeschichteter Aluminiumfolie enthaltend je 1 ml einer 0,2%igen Lösung von Evans Blue in phosphatgepufferter Kochsalz-Lösung,
Schraubdeckelflasche mit 100 ml phosphatgepufferter Kochsalzlösung, Anleitung zur Durchführung des Färbeverfahrens.

**Patentansprüche**

1. Verfahren zum Nachweis von Pilzen und Algen, dadurch gekennzeichnet, dass tierisches oder menschliches Gewebe oder Körperflüssigkeiten mit einem optischen Stilben-Aufheller mit mindestens 4 Sulfogruppen oder einem Salz davon behandelt und mit einem FluoreszenzMikroskop untersucht werden.

2. Verfahren gemäss Anspruch 1, dadurch gekennzeichnet, dass mit einem optischen Stilben-Aufheller mit 4 bis 6 Sulfogruppen oder einem Salz davon behandelt wird.

3. Verfahren gemäss Anspruch 1, dadurch gekennzeichnet, dass mit einem optischen Aufheller der Formel

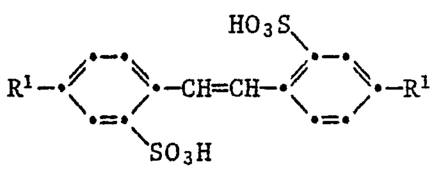

(I),

worin $R^1$ ein aromatischer oder heteroaromatischer Substituent, der mindestens eine Sulfogruppe trägt, oder eine durch einen aromatischen oder heteroaromatischen Rest mit mindestens einer Sulfogruppe substituierte Aminogruppe bedeutet und die beiden Reste $R^1$ gleich oder verschieden sein können, oder der Formel

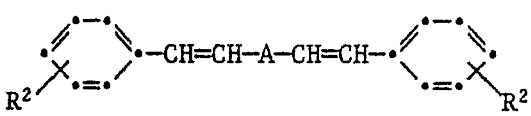

(II),

worin A ein durch Sulfogruppen substituierter 1,4-Phenylen- oder 4,4'-Biphenylylen-Rest und $R^2$ Sulfo, Hydroxy, Niederakoxy, Hydroxyniederalkoxy, Halo, Cyano, Carboxy, Niederalkoxycarbonyl oder Carbamoyl bedeutet oder die Bedeutungen von $R^1$ hat und die Reste $R^2$ gleich oder verschieden sein und auch mehrfach vorkommen können, oder einem Salz davon behandelt wird.

4. Verfahren gemäss Anspruch 1, dadurch gekennzeichnet, dass mit einem optischen Aufheller der Formel

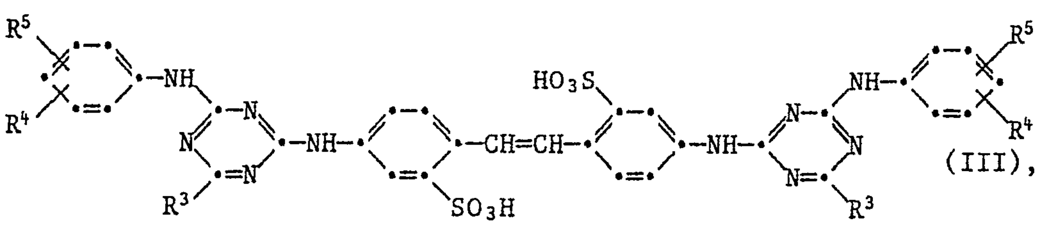

(III),

worin $R^3$ Hydroxy, Niederalkoxy, Hydroxyniederalkoxy, Niederalkoxyniederalkoxy, Niederalkylamino, Diniederalkylamino, Hydroxyniederalkylamino, Niederalkyl-hydroxyniederalkyl-amino, Di(hydroxyniederalkyl)amino, Niederalkoxyniederalkylamino, Niederalkyl-niederalkoxyniederalkyl-amino, Di(niederalkoxyniederalkyl)amino, Morpholino oder Phenylamino, das gegebenenfalls im Phenylring durch Sulfogruppen substituiert ist, $R^4$ Sulfo, $R^5$ Wasserstoff, Sulfo oder einen annellierten Phenylring bedeuten und jeweils die beiden Reste $R^3$, $R^4$ und $R^5$ gleich oder verschieden sein können, oder mit einem wasserlöslichen

Salz davon behandelt wird.

5. Verfahren gemäss Anspruch 1, dadurch gekennzeichnet, dass mit einer Verbindung der Formel III, worin $R^3$ Hydroxyniederalkylamino, Di(hydroxyniederalkyl)amino oder durch Sulfo substituiertes Phenylamino, $R^4$ Sulfo und $R^5$ Wasserstoff, Sulfo oder ein annellierter Phenylring bedeuten, oder mit einem wasserlöslichen Salz davon behandelt wird.

6. Verfahren gemäss Anspruch 1, dadurch gekennzeichnet, dass mit einer Verbindung der Formel III, worin $R^3$ Di(2-hydroxyethyl)amino oder 3-Sulfophenylamino, $R^4$ 3-Sulfo und $R^5$ Wasserstoff bedeuten, oder mit einem wasserlöslichen Salz davon behandelt wird.

7. Verfahren gemäss Anspruch 1, dadurch gekennzeichnet, dass mit 4,4'-Bis[2-di(2-hydroxyethyl)amino-4-(3-sulfophenylamino)-1,3,5-triazin-6-ylamino]-stilben-2,2'-disulfonsäure Natriumsalz behandelt wird.

8. Verfahren gemäss einem der Ansprüche 1 bis 7 zur Anfärbung von Pilzen und Algen in menschlichem Gewebe oder Körperflüssigkeiten.

9. Präparate zur Verwendung in einem Verfahren zum Nachweis von Pilzen und Algen enthaltend einen optischen Stilben-Aufheller mit mindestens 4 Sulfogruppen oder ein Salz davon und ein Trägermaterial.

10. Präparate gemäss Anspruch 9 in Form von wässrigen Lösungen.

11. Präparate gemäss Anspruch 9 in Form von wässrigen Lösungen enthaltend zwischen 0,05 % und 5 % des optischen Stilben-Aufhellers und Salze, organische Puffer, organische Lösungsmittel und/oder fixierende Stoffe.

12. Präparate gemäss einem der Ansprüchen 9, 10 oder 11 enthaltend einen optischen Aufheller der Formel

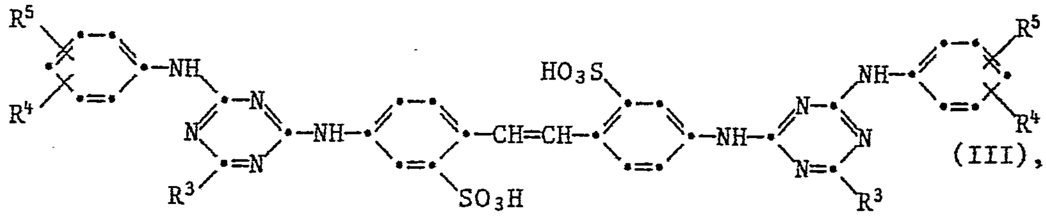

(III),

worin $R^3$ Hydroxy, Niederalkoxy, Hydroxyniederalkoxy, Niederalkoxyniederalkoxy, Niederalkylamino, Diniederalkylamino, Hydroxyniederalkylamino, Niederalkyl-hydroxyniederalkyl-amino, Di(hydroxyniederalkyl)amino, Niederalkoxyniederalkylamino, Niederalkyl-niederalkoxyniederalkyl-amino, Di(niederalkoxyniederalkyl)amino, Morpholino oder Phenylamino, das gegebenenfalls im Phenylring durch Sulfogruppen substituiert ist, $R^4$ Sulfo, $R^5$ Wasserstoff, Sulfo oder einen annellierten Phenylring bedeuten, oder ein wasserlösliches Salz davon.

13. Präparate gemäss einem der Ansprüche 9, 10 oder 11 enthaltend eine Verbindung der Formel III, worin $R^3$ Hydroxyniederalkylamino, Di(hydroxyniederalkyl)amino oder durch Sulfo substituiertes Phenylamino, $R^4$ Sulfo und $R^5$ Wasserstoff, Sulfo oder einen annellierten Phenylring bedeuten, oder ein wasserlösliches Salz davon.

14. Präparate gemäss einem der Ansprüche 9, 10 oder 11 enthaltend eine Verbindung der Formel III, worin $R^3$ Di(2-hydroxyethyl)amino oder 3-Sulfophenylamino, $R^4$ 3-Sulfo und $R^5$ Wasserstoff bedeuten, oder ein wasserlösliches Salz davon.

15. Präparate gemäss einem der Ansprüche 9, 10 oder 11 enthaltend 4,4'-Bis[2-di(2-hydroxyethyl)amino-4-(3-sulfophenylamino)-1,3,5-triazin-6-ylamino]-stilben-2,2'-disulfonsäure Natriumsalz.

16. Verfahren zur Herstellung von Präparaten zur Verwendung in einem Verfahren zum Nachweis von Pilzen und Algen, dadurch gekennzeichnet, dass man einen optischen Stilben-Aufheller mit mindestens 4 Sulfogruppen oder ein Salz davon mit einem Trägermaterial mischt.

17. Test-Packung enthaltend einen optischen Stilben-Aufheller mit mindestens 4 Sulfogruppen oder ein Salz davon zur Verwendung in einem Verfahren zum Nachweis von Pilzen und Algen in tierischem oder menschlichem Gewebe oder Körperflüssigkeiten.

18. Test-Packung gemäss Anspruch 17 enthaltend einen optischen Stilben-Aufheller mit mindestens 4 Sulfogruppen oder ein Salz davon und einen Farbstoff und/oder serologische Testlösungen und/oder eine Anleitung zur Durchführung des Verfahrens.

19. Test-Packung gemäss Anspruch 17 oder 18 enthaltend einen optischen Stilben-Aufheller in Form eines Präparates gemäss einem der Ansprüche 9 bis 15.

20. Test-Packung gemäss einem der Ansprüche 17, 18 oder 19 enthaltend 4,4'-Bis[2-di(2-hydroxyethyl)amino-4-(3-sulfophenylamino)-1,3,5-triazin-6-ylamino]-stilben-2,2'-disulfonsäure Natriumsalz.

21. Verwendung eines optischen Stilben-Aufhellers mit mindestens 4 Sulfogruppen oder eines Salzes davon in einem Verfahren zum Nachweis von Pilzen und Algen in tierischen oder menschlichem Gewebe oder Körperflüssigkeiten.